# EUROPEAN PATENT SPECIFICATION

(11) **EP 2 349 249 B1**
(45) Date of publication and mention of the grant of the patent: **05.04.2017**
(21) Application number: 09795546.2
(22) Date of filing: 15.10.2009
(51) Int. Cl.: A61K 31/198, A61K 31/7004, A61K 9/14, A61P 9/10

(54) **SYNERGISTIC COMPOSITION FOR RECOVERY AND REDUCTION OF MILD ISCHEMIC DAMAGE**
SYNERGISTISCHE ZUSAMMENSETZUNG ZUR ERHOLUNG UND MINIMIERUNG VON LEICHTEN ISCHÄMISCHEN LÄSIONEN
COMPOSITION SYNERGIQUE POUR RÉTABLIR ET RÉDUIRE UNE LÉSION ISCHÉMIQUE BÉNIGNE

(30) Priority: 15.10.2008 IT MI20081830
(43) Date of publication of application: 03.08.2011
(73) Proprietor: Giellepi S.p.A., 20123 Milano (IT)
(72) Inventor: TERRUZZI, Carlo, I-22030 Caglio (IT); LUCCHINA, Franco, I-21052 Busto Arsizio (IT); CARETTI, Anna, I-20142 Milan (IT)
(74) Representative: Zaccaro, Elisabetta
(86) International application number: PCT/IB2009/054534
(87) International publication number: WO 2010/044070

(56) References cited:
- WO-A-99/65476
- WO-A1-02/09727
- WO-A1-2007/146086
- US-B1- 6 172 114
- Anonymous: "Creatina + Ribosio" 31 October 2003 (2003-10-31), XP002585918 Retrieved from the Internet: URL:http://www.bodyweb.com/forums/integrat ori-uso-e-caratteristiche/52144-creatina-r ibosio.html> [retrieved on 2010-05-18]
- Anonymous: "Ribosio e creatina" 3 May 2001 (2001-05-03), XP002585919 Retrieved from the Internet: URL:http://www.bodyweb.com/forums/integrat ori-uso-e-caratteristiche/3025-ribosio-e-c reatina.html> [retrieved on 2010-05-18]
- Anonymous: "Ribosio + Creatina monoidrato" 7 August 2008 (2008-08-07), XP002585920 Retrieved from the Internet: URL:http://www.fituncensored.com/forums/in tegrazione/5134-ribosio-creatina-monoidrat o.html> [retrieved on 2010-05-18]

## Description

### Field of the invention

The present invention relates to a synergistic contribution of two substances to overcoming conditions that cause mild hypoxia in heart muscle, for instance in the post-infarcted heart and in conditions of coronary dysfunction, obstructive chronic pulmonary disease, mild myocardial ischemia.

### State of the art

Various pathologies may be associated with cellular hypoxia and ischemia caused by reduced blood and oxygen delivery. For example, several studies reported that insufficient cellular oxygenation is correlated with the degree of tumor aggressiveness and growth. Mild cerebral ischemia causes neuro-inflammatory responses involving the release of cytokines and nitric oxide. Metabolic and hematological disorders, sepsis, hypotension and pulmonary conditions may act as contributing factors in the onset of hypoxia and perinatal cyanosis.

Moreover, conditions that are direct consequence of myocardial ischemia and the often time-consuming and incomplete recovery of post-infarction patients are well known factors influencing health care expenses.

The individual use of the substances of interest in cardiovascular diseases is well known in the literature; in particular, it is well known that D-ribose, i.e. the sugar moiety of ATP, increases the tolerance to ischemia and ameliorates symptoms of many heart and muscle pathologies, although, under physiological conditions, this substance by itself has no specific effects on muscle strength and performance. The effects of creatine (Cr) administration to ameliorate the symptoms of many cardiac and muscle diseases, by increasing the size of muscle fibers and enhancing their performance (1) are also very well known. At least in part, such activity is due to the fact that creatine is an amino acid with an essential role in the regulation of energy metabolism in muscle: in presence of ATP it is converted to phosphocreatine and thus constitutes a cellular energy store.

Therefore, the combined administration of both compounds and their association with other molecules was never considered especially beneficial so far. For example, WO 02/09727 describes a composition including ribose and other food supplements, as for instance vitamins, folic acid etc., a composition which might as well include also creatine, pyruvate, taurine, or glutamine. Those compositions have generic ameliorating effects on various metabolic pathways, due to their increased supply of energy.

U.S. 6,172,114 also describes a nutritional supplement including creatine and ribose; in this context, however, creatine is provided in order to increase body mass and ribose is provided in order to counter major side effects of creatine, such as nausea and diarrhoea.

A synergistic effect essential for rescuing cell cycle progression in myocardial cells subjected to mild ischemia, as described in the present patent application, was never described before.

### Summary of the invention

The present invention is based on the observation that an extemporaneous preparation containing, as active ingredients, creatine and ribose shows synergistic activity in reducing mild ischemic damage and in promoting recovery.

Thus, according to a preferred embodiment the invention relates to a kit for preparation of an extemporaneous mixture comprising creatine and ribose and comprising one container of D-ribose and one container of creatine, together with the appropriate instructions for mixing the above substances in the proper weight ratio, ranging between 10:1 and 1:1 (D-ribose: creatine).

### Brief description of the figures

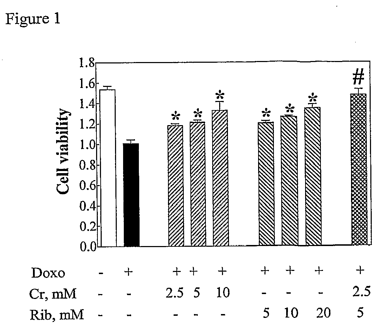

**Figure 1****.** The effect of increasing Cr and Rib concentrations, individually or in combination, on viability of doxorubicin-treated H9c2 cells. Cell viability determined by the MTT assay is expressed (mean±SEM, n = 6) versus the cell viability determined at *zero* time. ANOVA P<0.0001. *, P<0.01 vs doxorubicin-treated cells; #, P<0.001 vs cells treated with doxorubicin and with 2.5 mM Cr or 5mM Rib (Bonferroni post-test).

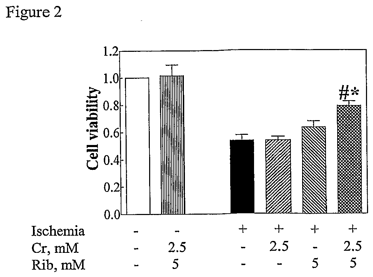

**Figure 2****.** Effect of the administration of Cr or Rib, and of their combination, on viability of H9c2 cells subjected to mild ischemia. Cell viability determined by the Trypan blue assay is expressed (mean±SEM, n = 6) versus the cell viability determined in untreated, non-ischemic cells. ANOVA P<0.0001. *, P<0.001 vs untreated ischemic cells; #, P<0.05 vs ischemic cells treated with both 2.5 mM Creatine and 5mM Ribose (Bonferroni post-test).

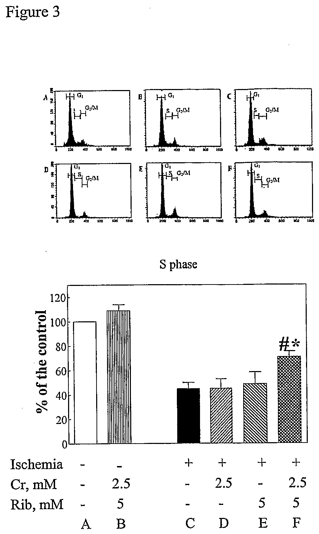

**Figure 3****.** Effect of the administration of Cr or Rib, and of their combination, on cell cycle progression in H9c2 cells subjected to ischemia. The top panel shows a typical diagram representative of the cell cycle; A-F show the various treatments detailed in the table. The lower panel shows, for each treatment, the percentage of cells in S phase (mean±SEM, n = 4) versus untreated non-ischemic cells. ANOVA P<0.0001. *, P<0.01 vs untreated ischemic cells; #, P<0.05 vs ischemic cells treated with both 2.5 mM Creatine and 5mM Ribose (Bonferroni post-test).

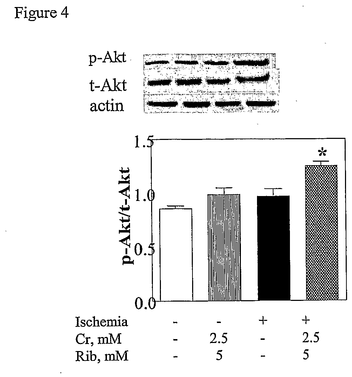

**Figure 4****.** Effect of combined administration of Cr and Rib on the expression of phosphorylated (p-) and total (t-) Akt protein in H9c2 cells subjected to ischemia. Western blot images are representative of the content in both phosphorylated and total Akt proteins. The p-Akt/t-Akt ratio (mean±SEM, n = 4) was determined by densitometric analysis of protein bands, as shown in the graph below. ANOVA P<0.002. *, P<0.05 vs untreated ischemic cells (Bonferroni post-test).

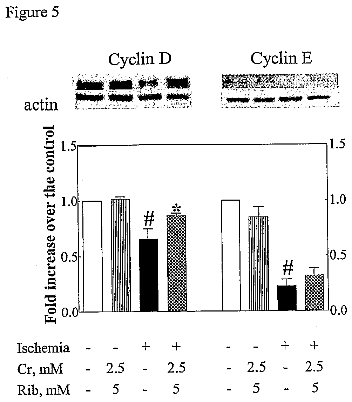

**Figure 5****.** Effect of the combined administration of creatine and ribose on expression of cyclin D and E proteins in H9c2 cells subjected to ischemia. Western blot images are representative of the content in cyclin D and E proteins, actin is used as control for equal sample loading. The graph below shows the corresponding densitometric analysis of protein bands (mean±SEM, n = 4). Data are expressed as variation from the value determined in non-ischemic untreated cells. ANOVA P<0.001; *, P<0.05 vs ischemic untreated cells; #, P<0.01 vs non-ischemic untreated cells (Bonferroni post-test) for cyclin D. ANOVA P<0.0001; #, P<0.001 vs non-ischemic untreated cells (Bonferroni post-test) for cyclin E.

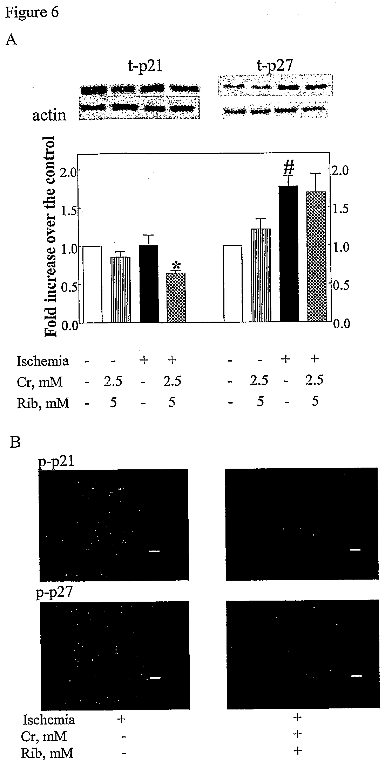

**Figure 6****.** Effect of the combined administration of creatine and ribose on the expression of the cyclin-dependent kinase inhibitor proteins, p21 and p27, in H9c2 cells subjected to ischemia. Panel A: Western blot images are representative of the total content in p21 and p27 proteins, actin is used as control for equal sample loading. The graph below shows the corresponding densitometric analysis of protein bands (mean±SEM, n = 4). Data are expressed as variation from the value determined in non-ischemic untreated cells. ANOVA P=0.02; *, P<0.05 vs untreated ischemic cells (Bonferroni post-test) for p21. ANOVA P=0.002; *, P<0.01 vs untreated ischemic cells (Bonferroni post-test) for p27. Panel B: immunofluorescence images representative of the phosphorylated forms of p21 and p27 in H9c2 cells subjected to ischemia, in presence or absence of Cr and Rib. 40x magnification, the bar corresponds to 20 µm.

### Detailed description of the invention.

In a first aspect, the present invention provides a kit for extemporaneous preparation of a mixture comprising creatine and ribose, as described in claims 1 to 9.

In a further aspect, the present invention provides a the use of an extemporaneous composition of D-ribose and creatine in a ratio comprised from 10:1 to 1:1 (w/w) for reducing and promoting recovery from mild ischemic damage. The present invention is based on the observation that an extemporaneous preparation containing, as active ingredients, creatine and ribose shows synergistic activity in reducing mild ischemic damage and in promoting recovery, thereby restoring viability and reactivating the cell cycle after a mild ischemic event.

In fact, using *in vitro* models, it has been now discovered that when these two substances are combined immediately before their addition to isolated cells, extemporaneous mixing of the two substances results in a measurable synergistic effect in the cellular model selected for the study: in fact, using specific cellular markers, the re-entry into cell cycle of cells undergone mild ischemic damage is observed along with reduced necrosis of the surrounding tissue. Under *in vivo* conditions of mild ischemia caused by hypoxia, this would decrease the extent of permanent damage caused by an ischemic event.

However, the combined storage of these two substances raises issues concerning their stability.

Therefore, the present invention solves the technical problem related to storage of the two substances in appropriate doses and in a ratio suitable for their administration, since the two substances are mixed immediately before use, thereby allowing the synergistic effect to occur, since such effect results exclusively from their extemporaneous mixing.

Therefore a first use of the invention is for mild ischemic tissue damage, particularly in the myocardium, represented by the cellular model of H9c2 cardiomyocytes grown *in vitro* and subjected to hypoxic conditions corresponding to 1% partial O₂ pressure, in the absence of glucose.

Due to inherent characteristics of the model used, it was possible to observe that both creatine and ribose owe to be mixed immediately before use for cellular functions to be restored following the application of such conditions.

Cell viability and proliferation were assessed in the H9c2 cardiomyocyte model following ischemia induced by hypoxia upon exposure to 1% O₂ combined with glucose deprivation. Contrary to the events caused by more severe hypoxic conditions, the resulting cellular damage is potentially reversible and the reversal is positively associated with an increase of cell cycle progression of cardiomyocytes.

At the cellular level, under conditions of mild hypoxia, the decrease in expression or the activation of some cell cycle regulatory proteins (11, 13) was found to be less dramatic if both creatine and D-ribose were present in the culture medium after they are mixed immediately before their addition to the medium. The effect of the two substances on the cell cycle is greater than the sum of their individual effects, therefore showing features of a synergistic interaction.

In particular, the following parameters were measured in the cellular model: the level of phosphorylation of the Akt serine-threonine kinase (protein kinase B), which is well-known to induce cell cycle progression (12) via stimulation of cyclins and inhibition of cyclin inhibitors, and the level of expression of cell cycle regulatory proteins (cyclin D and p21) which are indicators of cell viability, proliferation and regeneration.

The observed effect of combined treatment, as described in detail in the experimental part, was detected at the molecular level as an increase of Akt phosphorylation that was particularly evident in cells subjected to ischemia (Figure 4) but was also present at a low level in normal cells.

As better described in the experimental part, when the activation of some transducers of Akt phosphorylation was measured it was found that, compared to a control subjected to mild ischemia, the level of cyclins (especially cyclin D) increased and the level of cyclin inhibitors (especially t-p21) decreased.

In the essence, the effect of combined treatment with creatine and ribose on ischemic cardiomyocytes interferes positively with cellular signaling pathways involved in cell cycle progression and is more effective than the individual treatment with each one of such substances.

A combined effect of both substances on cell viability is also detectable. Indeed, following an ischemic stimulus that decreases cell viability by 50±4%, the presence of both substances, but not of each substance alone, increases this parameter by about 30±3%.

Thus, simultaneous administration of creatine and ribose to H9c2 cells subjected to ischemia results in an increase of viability up to a level that is even comparable to cells not subjected to the ischemic stimulus. Instead this treatment is ineffective in non-ischemic cells.

The effect on viability detected in cardiomyocytes involves cell cycle regulation: the ischemia-induced cell cycle arrest is decreased in presence of both creatine and D-Ribose due to the activation of Akt and its mediators, in particular a cyclin inhibitor, known as t-p21 (9), and cyclin D (12).

In fact, while the hypoxia is a well-known inducer of cell cycle arrest at the level of G1/S transition in various cell lines (5) and the Akt protein kinase (PI3K-Akt, 8) is a well-known and essential modulator of cell cycle via a protective signaling pathway, this study has shown that creatine and ribose, only when they are simultaneously present, act synergistically to reverse, via PI3K-Akt, the process of ischemia-induced cyclin inhibition and that this effect is particularly strong on cyclin D.

Without being linked to a particular technical explanation for this effect, the complementarity of creatine and ribose in exerting such effect may account for the especially beneficial effect of combined administration of these two substances, which is at the basis of the present invention: indeed the accumulation of creatine in skeletal muscle and in cardiomyocytes, a phenomenon that was already observed in human subjects after oral intake of creatine, requires a Na-Cr (sodium-creatine) co-transporter. Since the activity of this co-transporter is insulin-dependent, it may be possibly enhanced by coadministration of sugars such as ribose.

However, the preparation of doses containing a mixture of the two substances in a form that is ready for administration involves an issue related to the storage of creatine, owing to the fact that this substance has a tendency to decompose in the presence of a weak reducing agent like D-ribose.

To solve this technical issue, the applicant has identified some preferred embodiments, under the form of various types of kits, wherein the two substances are combined, preferably immediately before use, and the final ratio D-ribose to creatine is comprised between 10:1 and 1:1 and more preferably between 8:1 and 2:1, and even more preferably is approximately 5:1.

In particular, according to an embodiment, creatine is prepared in the form of powder or granulates, preferably in presence of a lubricating agent suitable to facilitate the pre-packing flow and dosage; more preferably an anti-agglomerating agent, such as silicon dioxide, is also added. D-ribose can be prepared in predosed form, both in the form of powder or in a form dissolved in aqueous solution in presence of preserving agents such as, for example, benzoic acid derivatives and/or potassium sorbate in a maximum amount of 0.2%. The powder is mixed in solution immediately before administration.

In the embodiment wherein both creatine and D-ribose are in the form of powder, these two substances can be stored already as mixture in the form of powder or granulates, and the mixture can be placed in pre-dosed sachets containing both molecules in a weight ratio comprised between 10:1 and 1:1, more preferably between 8:1 and 2:1, even more preferably in a ratio of approximately 5:1.

This preparation is preferably made by pre-mixing creatine with a lubricating agent, such as magnesium stearate, in a weight ratio of about 1:100, or in a ratio that is well known to the expert in the field, while D-ribose is pre-mixed with an anti-aggregating agent, such as Silicon dioxide, or with another anti-aggregating agents with similar characteristics, such as calcium phosphate or colloidal silica, in a ratio of approximately 1:200, however in an amount not greater than 5%, or preferably 1%.

Under these conditions, the final mixture, in the above mentioned ratio, is stable for at least 24 months at temperature not exceeding 25 °C.

Furthermore according to this embodiment, the single-dose sachet preferably contains approximately 6.05 g of mixture, consisting of 5 g of ribose and 1 g of creatine. Multiple doses or fractions of the material in this optimal ratio can be made, depending on the minimum amount of powder that can be dosed by mechanical means.

Such preferred ratios and the optimal dose take into account the solubility of each of the two molecules, preferably in a volume of approximately 100 ml of aqueous solution or buffer.

According to another embodiment, the two powders can be pre-dosed according to the above defined weight ratios and kept in separate but entwined sachets to be mixed and dissolved in water or in aqueous solution immediately before use.

A further aspect of this embodiment is represented by a liquid preparation wherein D-ribose is dissolved at concentrations varying between 10 and 80% (w/w) in presence of preserving agents, such as benzoic acid derivatives and/or potassium sorbate, and powdered creatine is instead dosed in a reservoir cap according to the same weight ratios to creatine mentioned above.

From the above, it follows that the invention therefore comprises a kit for preparation of a mixture comprising creatine and ribose for use in ameliorating mild ischemic damage, and such kit includes a container with D-ribose and a container with creatine together with appropriate instructions for mixing immediately before administration in order to achieve the optimal activity discovered for the mixture, and in order to maintain a proper weight ratio between the two substances that is comprised between 10:1 and 1:1 (D-ribose: creatine). According to an embodiment of the invention, the kit comprises D-ribose in the form of powder and, according to this realization, the powdered D-ribose is preferably mixed with an anti-agglomerating agent, such as silicon dioxide or its analogues, in amounts not greater than 5%, preferably 1%. According to an alternative embodiment, the kit contains creatine in the form of powder and D-ribose in solution, preferably in presence of preservatives (e.g. benzoic acid derivative and potassium sorbate).

The kit can also be suitably realized as powder mixture of D-ribose, and creatine, wherein the weight ratio between the two substances is comprised between 10:1 and 1:1, and such mixture, contained in a suitable container, box or sachet, is to be used for the treatment of mild ischemic damage, and is provided together with appropriate instructions for solubilization of the mixture immediately before administration. Preferably, such mixture also includes a lubricating agent and an anti-agglomerating agent, as identified above, wherein the lubricating agent is preferably magnesium stearate and the anti-agglomerating agent is preferably silicon dioxide.

According to further embodiments, the invention further comprises: instructions for preparation of a stable mixture of D-ribose and creatine, involving the mixing of creatine, with a lubricating agent and the mixing of D-ribose, with an anti-agglomerating agent.

Moreover, the invention relates to the association of such substances in a composition of D-ribose and creatine in a weight ratio comprised between 10:1 and 1:1 (w/w), for use in prevention or reduction of myocardial ischemic damage caused by conditions of mild hypoxia, wherein the reduced ischemic damage is associated with increased cell cycle progression of cardiomyocytes that is induced in a synergistic manner by treatment with the compositions according to the invention.

### EXPERIMENTAL PART

### Materials and methods. Materials.

Creatine monohydrate and D-ribose (Giellepi Chemicals, Milan, Italy) were dissolved in culture medium, filtered through a 0.22 µm filter (Nalgene) and stored at 4°C. The H9c2 cardiomyocyte cell line was ordered from the American Type Culture Collection (Rockville, MD). Cells were maintained in a 37°C incubator in 5% CO₂ atmosphere, in Dulbecco's modified Eagle's medium supplemented with 10% inactivated foetal calf serum, 100 units/ml penicillin and 100 µg/ml streptomycin. Cells were sub-cultured by 1:3 dilution every three days, using 25 cm² flasks.

After reaching 80% confluency, cells were subjected to the different treatments, using in each case n= 4-6 cellular pools.

### Cell viability assay: MTT.

Cells were seeded at a concentration of 1.8x10³/well in 96-well plates and incubated at 37°C for 24 hours. Cells were then incubated for additional 24 hours after addition of water or doxorubicin (0.5 µM final concentration) in presence of different concentrations of Cr and/or Rib, as indicated in the figures. Cell viability was determined by the 2-(4,5-dimethyltriazol-2-yl)-2,5-diphenyl tetrazolium bromide assay (MTT, Sigma, St Louis, Mo). Briefly, after addition to each well of 20µl of the MTT solution in PBS at a concentration of 5 mg/ml, cells were incubated at 37°C for 4 hours. After removal of the supernatant, 100 µl of DMSO were added to dissolve the formazan salts produced and optical density (OD) was read at λ=540 nm. For each treatment, five replicates were performed. The viability index was calculated, for each treatment, as ODx/OD₀, where ODx and OD₀ are the average ODs at time x and at time zero.

### Trypan blue exclusion test.

Viable cells were counted by the Trypan blue exclusion test. An aliquot of trypsinized cells was mixed with the dye and observed under the microscope for counting viable cells. Data were expressed as percentage of viable cells following the various treatments compared with control cells (which were set equal to 100) maintained under standard conditions for the same time length.

### Model of simulated ischemia.

Cells were seeded in 25 cm² flasks at a concentration of 3.5x10⁵ cells/flask. Next, the model of simulated ischemia (11, 13) involved a step wherein cell flasks were saturated for 30 min in 1% O₂ atmosphere (a condition of mild hypoxia) using medium lacking glucose (Gibco, Invitrogen, #11966), in presence of Creatine and/or D-ribose or of these two substances in combination that were mixed at the same time as the individual treatments with single dose The mixing in a 1:2 ratio was made immediately before addition to culture medium, as specified in each figure, followed by sealing of the flasks (ischemic cells) that were kept sealed for the following 24 hours.

### Flow cytometry

The cell cycle profile was analyzed by flow cytometry after DNA staining with propidium iodide (PI, Sigma, St. Louis, Mo.). Cells were washed with PBS and fixed with 70% ethanol at -20°C, centrifuged (15 min at 1800 rpm), resuspended in 1 ml PBS containing 50 µg/ml PI and incubated for 1 hour at room temperature Cells were then analyzed by flow cytometry in a FACSorter (Becton Dickinson). The percentage of cells in different phases of the cell cycle was calculated using the CellQuest software program (Becton Dickinson).

### Immunoblotting.

Cells were twice washed with cold PBS and incubated for 30 minutes in lysis buffer (50 mM Tris-HCl pH 7.4, 250 mM NaCl, 1% Triton X-100, 50 mM β-glycerol phosphate, 1 mM Na₃VO₄, 1 mM NaF, 1 mM phenyl methyl sulfonyl fluoride, Sigma, St. Louis, Mo.) containing 2% Protease inhibitor cocktail (Complete, EDTA-free, Roche Diagnostics, Germany). Cells subjected to simulated ischemia were kept in a suitable compensation chamber ensuring low O₂ tension during the extraction procedure (2).

The cell lysates thus obtained were frozen at -20°C for 20 minutes, thawed at room temperature and centrifuged at 14,000 rpm for 15 min at 4°C. Protein concentration was determined by the Coomassie Plus Protein Assay reagent Kit (Pierce). Forty µg of total proteins were applied to each gel well. After electrophoretic separation, proteins were transferred to a nitrocellulose membrane (Amersham Pharmacia Biotech, Little Chalfont, Buckinghamshire, UK) that was then treated for 1 hour with 5% milk to block non-specific binding sites, incubated overnight at 4°C with primary antibody, followed by incubation with secondary antibody for 1 hour at room temperature. The following antibodies were used: anti-phospho-Akt polyclonal (Ser473, Cell Signaling, 1:1000), anti-Akt polyclonal (Cell Signaling, 1:1000), anti-cyclin D1 polyclonal (Santa Cruz, 1:750), anti-cyclin E monoclonal (Santa Cruz, 1:750), anti-actin polyclonal (Cell Signaling, 1:1000), anti-phospho-p21 polyclonal (Thr145, Santa Cruz, 1:500), anti-p21 polyclonal (Santa Cruz, 1:1000), anti-phospho-p27 polyclonal (Thr187, Santa Cruz, 1:400), anti-p27 polyclonal (Santa Cruz, 1:750). IgG secondary antibodies were peroxidase-conjugated anti-mouse (Jackson Immuno Research, West Grove, PA, 1:10000), rabbit (Jackson Immuno Research, West Grove, PA, 1:10000) or goat (Southern, Biotech, 1:10000). Staining of actin was used to control the amount of loaded proteins.

After incubation of the membrane for 1 minute with the LiteAblot Chemiluminescent kit (Lite Ablot, EuroClone, EMPO10004), photographic films (Kodak X-Omat Blue XB-1 Film, Eastman Kodak Company, Rochester, NY) were exposed to the chemiluminescent signal. For quantitative assessment of band intensities, the image was acquired and quantified by the Gel Doc apparatus, using the Quantity One (Bio-Rad) densitometric analysis program.

### Immunofluorescence

Cells were centrifuged (800 rpm for 5 min at room temperature), twice washed in PBS, centrifuged again (1000 rpm for 5 min at 4°C) and fixed in 5% formalin for 30 minutes at 4°C. After a further centrifugation (1000 rpm for 5 min at 4°C), cells were placed on silanized slides, post-fixed for 5 minutes at -20°C with ethanol-acetic acid 2:1, twice washed with PBS and boiled in 10mM citrate buffer pH 6.0 for 10 minutes. Samples were then treated with 10% serum for 1 hour, incubated overnight at 4°C with primary antibody followed by 45 minute incubation with secondary antibody conjugated to fluorescein (Santa Cruz, 1:200) or rhodamine (Invitrogen, 1:200). For each sample, a negative control was obtained by replacing the primary antibody with 1 .5% normal serum. Images were acquired as previously described (4).

### Statistical Analysis.

All data were expressed as mean±SEM. Statistical analysis using one-way ANOVA followed, when it was significant (P <0.05), by the Bonferroni post-test, allowed to assess the significance of the differences between treatment groups.

### Example 1. Defining the optimal concentrations of Creatine e Ribose.

The optimal working concentration was determined by a dose-response curve using the MTT assay (vital dye staining) in presence of Creatine and Ribose. To test the efficacy of creatine and ribose in maintaining cellular energy homeostasis, H9c2 cells were treated with doxorubicin, a well-known cardiotoxic agent that causes mitochondrial damage resulting in production of reactive oxygen species. The concentration range selected for the *in vitro* system was around 5 mM for Creatine, an intermediate value in dose-response curves, and was around 10mM for D-ribose, corresponding to a 1: 2 weight ratio.

Also in the model of mitochondrial damage that was used (treatment with doxorubicin associated with mitochondrial damage and production of reactive oxygen species), the combined administration of creatine and ribose was more effective than each substance alone in improving viability of H9c2 cells that were subjected to treatment.

The minimum effective concentration of creatine and ribose required to improve viability of H9c2 cells treated for 24 hours with 0.5 µM doxorubicin (Doxo) was determined by the dose-response curve shown in Figure 1. Doxo decreased viability by 35% compared to untreated cells (P <0.001) Administration of creatine or Ribose increased cell viability in a dose-dependent manner : in particular, Creatine at 2.5 mM concentration and D-Ribose at 5 mM concentration were sufficient to induce protection.

Moreover, combined administration of creatine and ribose increases the cell viability index as compared to either substance alone, in agreement with what can be deduced in Figure 1 from the model of mitochondrial stress that was used.

### Example 2. Defining the model of simulated ischemia: effect of the combined administration of Creatine and Ribose on cell viability.

The model of simulated ischemia involves 24 hour incubation of cells at 37°C in medium lacking glucose, in an environment with low oxygen tension (11). Viability of ischemic cells, analyzed by the Trypan blue assay, decreased by 50±4% compared to non-ischemic cells (Figure 2). Compared to untreated ischemic cells, addition of 2.5 mM Creatine or 5 mM Ribose to the culture medium did not improve viability (P=NS). However, in presence of both substances, viability was increased by 30±3% compared to untreated cells. Viability was unchanged in control cells supplemented with 2.5 mM Creatine and 5 mM Ribose in combination (P=NS vs. control cells). Thus the viability index of H9c2 cells subjected to ischemia was increased by the simultaneous presence in the culture medium of creatine and ribose, but not by each substance alone. Instead the same treatment was ineffective in non-ischemic cells.

### Example 3. Assessment of the effects on ischemia-induced cell cycle arrest.

H9c2 cells subjected to ischemia showed a clear cell cycle arrest at S phase entry in (Figure 3). The percentage of cells progressing from G₁ to S phase was reduced by 55±4%. Compared to untreated ischemic cells, the administration of 2.5 mM Creatine or 5 mM Ribose individually did not change this percentage (P =NS). In contrast, combined administration the two substances increased the percentage of cells progressing from G1 to S phase by 25±4% compared to untreated non-ischemic cells. Other phases of the cell cycle were not found to be susceptible to the combined treatment with the two substances. Thus, the simultaneous administration of creatine and ribose, but not the administration of each of these two substances alone, attenuated the cell cycle arrest at the G1-S border. Whatever the treatment used, significant changes at the level of G0/G1 and G2/M transitions were not observed in ischemic cells.

### Example 4. Effects of creatine and D-Ribose on Akt phosphorylation level.

The content in the phosphorylated form of Akt, a well-known cell cycle modulator, was analyzed in the model of simulated ischemia. Figure 4 shows the Akt protein content, including both phosphorylated form (p-) and total protein (t), in H9c2 cells subjected to different treatments. This relationship was found not to change significantly upon ischemia, while the administration of creatine and ribose induces p-Akt (phosphorylated form), without changing the total Akt protein level. This results in increased p-Akt/t-Akt ratio, as indicated by densitometric analysis of protein bands (Figure 4). Such effect was not observed in non-ischemic cells. Thus, increased phosphorylation of Akt may in part explain the rescue from the cell cycle arrest that takes place following combined treatment with creatine and ribose.

### Example 5. Effect of combined administration of creatine and D-ribose on expression of cyclins and their inhibitors.

The level of cyclins D1 and E and of cyclin-dependent kinase inhibitors, p21 and p27, which are effectors of phosphorylated Akt in modulation of cell cycle progression from G₁ to S phase, were measured in H9c2 cells subjected to ischemia.

Analysis of protein expression showed that ischemia decreases cyclin D1 content (P <0.01 vs. non-ischemic cells, Figure 5) whereas the treatment with creatine and ribose promotes cyclin D1 synthesis (P <0.05 vs untreated ischemic cells). Instead the combined administration of creatine and D-ribose was rather ineffective in restoring the content in cyclin E protein, which was significantly reduced as result of ischemia (P = 0.001 vs non-ischemic cells). Finally, we examined whether the cell cycle inhibitors p21 and p27 mediate the effect of combined administration of Creatine and Ribose.

As result it was found that ischemia did not affect p21 expression, whereas the level of p27 was increased. Combined treatment with Creatine and Ribose slightly decreased p21 content but was ineffective on p27 expression (Figure 6A). Since expression of the phosphorylated forms of p21 and p27 proteins is very low and their determination by immunoblotting is therefore difficult, for semiquantitative analysis we resorted to immunofluorescence microscopy (Figure 6B). In ischemic cells, administration of creatine and ribose increased the phosphorylated form of p21, almost exclusively localized in the cytoplasm. The treatment did not affect the level of the phosphorylated form of p27, which was reduced by ischemia.

From an analysis of the various experiments carried out with H9c2 cardiomyocytes, it is possible to conclude that ischemia results in decreased cell viability associated with cell cycle arrest at the G1/S border.

Simultaneous administration of freshly premixed Creatine and Ribose, but not of each substance alone, induces a 30% increase of both cell viability and cell cycle progression. The mechanisms underlying the beneficial effects of a combination of Creatine and Ribose involve over-expression of proteins that are stimulatory to cell cycle progression and down-regulation of proteins that are inhibitory to cell cycle progression.

### Example 6. Extemporaneous preparation of compositions in the form of powder.

The compounds according to the invention were packaged in soluble granular form into bipartite sachets containing the two active substances separated one from the other.

The following amounts of the components required for preparation of an industrial batch of 35,000 sachets were weighed separately:
- D-Ribose 175 kg
- Creatine monohydrate 35 kg;
Since D-ribose is highly hygroscopic, the rules of good manufacturing recommend blending with silicon dioxide in the following proportions:

| | |
|---|---|
| - D-ribose | 99.9-98% |
| - Silicon dioxide (e.g. Aerosil 200) | 0.1-2%. |

Pre-weighed amounts of D-ribose and Aerosil 200 were placed into a biconical blender of 300 liter capacity for 15 minutes until reaching a good distribution of the silica in D-ribose. The resulting mixture shows good characteristics with respect to smoothness and low hygroscopic properties.

The mixture was discharged into a suitable container which was tightly sealed and placed in a special loading station of the sachet-filling machine.

After weighing an amount of very fine granular creatine monohydrate that is required for production of the industrial batch, this was discharged into a separate container suitable for loading the sachet-filling machine, which was tightly sealed. The SIG RPD type packaging machine was placed in a humidity control room in order to avoid moisture adsorption during packing operations. Humidity of the sachet dispensing room was maintained at HR not greater than 20%.

By use of suitable dispensers, the two components were dosed separately in sachets bearing an additional central welding: this way, the components of the formulation do not come in contact with each other.

The dispensers can be adjusted in order to dose, in the respective compartments, amounts ranging from 0.5g to 5g, thereby obtaining sachets containing all the quantities of the components included in this interval.

For example, possible combinations are:
a) D-ribose 5 g, Creatine 1 g; b): D-ribose 10 g + creatine 2 g; c) D-ribose 2:5 g + creatine 0.5 g.

Subsequently the filled, heat sealed sachets were rearranged and conveyed to the casing machine for secondary case packaging.

Separate dispensing of D-ribose and creatine components improves product stability over time and prevents possible browning reactions, the development of unpleasant odors and loss of activity of the individual components.

### Example 7. Preparations with D-ribose in liquid form.

The example of realization relates to compositions in a drinkable vial with dosing reservoir cap, realized as follows:
- solutions of D-ribose in purifed, demineralized water, in amounts varying between 10% (w/w) and 80% (w/w) taking into account D-ribose solubility, were prepared in the cold using a dissolver for liquids.
- solutions with various concentrations were prepared, and the aqueous solutions were stabilized by addition of preservatives such as benzoic acid derivatives and potassium sorbate in a maximum amount of 0.2% w/w.

The resulting solution was filled into vials by use of a suitable FARMOMAC vial-filling machine, using PET vials with volumes ranging from 5 ml to 30 ml.

Using a suitable machine for opercularization, the reservoir caps were filled with an amount of creatine monohydrate varying between 0.1 g and 1 g.

Reservoir caps are pressure inserted into the neck of the vials in order to achieve tight sealing of the vial.

Breakage of the membrane can be obtained by screwing the cap, thereby allowing creatine to mix with the solution containing ribose.

After vigorous agitation, the preparation is ready for administration.

For example, the following doses by weight were prepared:
- 10 ml vial: D-Ribose 7 g, creatine 0.5 g;
- 7 ml vial: D-Ribose 2.5 g, creatine 0.5 g;
- 15 ml vial: D-Ribose 10 g, creatine 1 g;

Other combinations of the two components are possible in the range comprised between 10:1 and 1:1, provided that D-ribose is always in solution and creatine is in solid form, due to solubility and stability issues arising from the fact that this substance is less soluble than D-ribose and is less stable in aqueous solution.

### Example 8. Preparation of a composition in granular form which is dispensed in twin sachets.

Separate preparations of the two components, D-ribose and Creatine, are made as already described in Example 5, maintaining the technical and operational solutions as well as the formulations described above.

The two components D-ribose and creatine, in their appropriate sealed containers, were positioned so as to feed the two dispensers of a Marchesini sachet-filling machine, RC model.

Partitioning in twin sachets was made separately, with the possibility to change the dose range for both components from 1g to 10g. This way, it was possible to dose higher amounts than those indicated in Example 5.

Possible combinations of D-ribose and creatine, within the above range, are the following:
a) D-ribose 10 g (Sachet A), creatine 2 g (Sachet B)
b) D-ribose 10 g (Sachet A), creatine 0.5 g (Sachet B)
c) D-ribose 5 g (Sachet A), creatine 1 g (Sachet B)

### Example 9. Preparation of a composition in granular form which is dispensed in twin sachets.

For preparation of a food supplement in the form of granular mixture which is dispensed in heat sealed sachets suitable for extemporaneous preparation in water.

It is well known that a reducing sugar like D-ribose reacts with -(NH2) amino groups under special conditions of humidity, pH and temperature, giving rise to darkening phenomena shifting toward a brown colour and to aromatic notes reminiscent of caramel flavour. This is known as the "Maillard" reaction and must be carefully kept under control during the preparation of foodstuff because it is an index of quality loss.

The mixture of D-ribose and creatine requires special precautions in order to avoid such reaction to occur.

Commercially available creatine is in monohydrate form with average loss on drying of about 12%. D-ribose, while having a very low humidity around 1%, on average, is very hygroscopic and tends to absorb moisture from both the environment and other components in a mixture.

The preparation of a stable mixture of creatine and D-ribose is possible if the following measures are applied:
450 Kg of D-ribose and 4.5 kg of silicon dioxide, AEROSIL® 200 or other equivalent product are placed into a stainless steel biconical blender of 1000 liter capacity, and intimately mixed for about 30'.

90 kg creatine monohydrate and 1.8 kg magnesium stearate, preferably of vegetal origin, are loaded into a second similar blender, of about 200 liters capacity, and mixed for approximately 30'.

The two resulting mixtures are then mixed together in a biconical or "V" blender of 1200 liters capacity for 15'. The final mixture is discharged into a tightly sealed container suitable for loading a sachet-filling machine for dispensing as single-dose sachets.

Dispensing is done by dosing approximately 6:05 g of mixture for each sachet, such that the content corresponds to 5 g of D-ribose and 1 g of creatine monohydrate. The mixture obtained under these conditions is stable for at least 24 months after preparation, provided it is stored at room temperature not exceeding 25°C.

Mixtures with different titers can be obtained by varying the amount of creatine monohydrate and D-ribose and, as described above, by varying in proportion to the amount of silicon dioxide and magnesium stearate excipients.

### Bibliography

1. Burke DG, Chilibeck PD, Parise G, Candow DG, Mahoney D, and Tarnopolsky M. Effect of creatine and weight training on muscle creatine and performance in vegetarians. Med Sci Sports Exerc 35: 1946-1955, 2003.
2. Corno AF, Milano G, Samaja M, Tozzi P, and von Segesser LK. Chronic hypoxia: a model for cyanotic congenital heart defects. J Thorac Cardiovasc Surg 124: 105-112, 2002.
3. Dunne L, Worley S, and Macknin M. Ribose versus dextrose supplementation, association with rowing performance: a double-blind study. Clin J Sport Med 16: 68-71, 2006.
4. Fantacci M, Bianciardi P, Caretti A, Coleman TR, Cerami A, Brines M, and Samaja M. Carbamylated erythropoietin ameliorates the metabolic stress induced in vivo by severe chronic hypoxia. Proc Natl Acad Sci U S A 103: 17531-17536, 2006.
5. Hammer S, To KK, Yoo YG, Koshiji M, and Huang LE. Hypoxic suppression of the cell cycle gene CDC25A in tumor cells. Cell Cycle 6: 1919-1926, 2007.
6. Hochachka P and Matheson G. Regulating ATP turnover rates over broad dynamic work ranges in skeletal muscles. J Appl Physiol 73: 1697-1703, 1992.
7. Hultman E, Soderlund K, Timmons JA, Cederblad G, and Greenhaff PL. Muscle creatine loading in men. J Appl Physiol 81: 232-237, 1996.
8. Maddika S, Ande SR, Panigrahi S, Paranjothy T, Weglarczyk K, Zuse A, Eshraghi M, Manda KD, Wiechec E, and Los M. Cell survival, cell death and cell cycle pathways are interconnected: implications for cancer therapy. Drug Resist Updat 10: 13-29, 2007.
9. Mullany LK, Nelsen CJ, Hanse EA, Goggin MM, Anttila CK, Peterson M, Bitterman PB, Raghavan A, Crary GS, and Albrecht JH. Akt-mediated liver growth promotes induction of cyclin E through a novel translational mechanism and a p21-mediated cell cycle arrest. J Biol Chem 282: 21244-21252, 2007.
10. Odoom JE, Kemp GJ, and Radda GK. The regulation of total creatine content in a myoblast cell line. Mol Cell Biochem 158: 179-188, 1996.
11. Patel HH, Tsutsumi YM, Head BP, Niesman IR, Jennings M, Horikawa Y, Huang D, Moreno AL, Patel PM, Insel PA, and Roth DM. Mechanisms of cardiac protection from ischemia/reperfusion injury: a role for caveolae and caveolin-1. FASEB J 21: 1565-1574, 2007.
12. Sale EM and Sale GJ. Protein kinase B: signalling roles and therapeutic targeting. Cell Mol Life Sci 65: 113-127, 2008.
13. Takahashi K, Ohyabu Y, Solodushko V, Takatani T, Itoh T, Schaffer SW, and Azuma J. Taurine renders the cell resistant to ischemia-induced injury in cultured neonatal rat cardiomyocytes. J Cardiovasc Pharmacol 41: 726-733, 2003.
14. Wallis J, Lygate CA, Fischer A, ten Hove M, Schneider JE, Sebag-Montefiore L, Dawson D, Hulbert K, Zhang W, Zhang MH, Watkins H, Clarke K, and Neubauer S. Supranormal myocardial creatine and phosphocreatine concentrations lead to cardiac hypertrophy and heart failure: insights from creatine transporter-overexpressing transgenic mice. Circulation 112: 3131-3139, 2005.

## Claims

1. Kit for extemporaneous preparation of a mixture comprising creatine and ribose, including at least one container with D-ribose, one container with creatine and appropriate instructions for solubilization and mixing, or vice-versa, of the two components, wherein such instructions relate to the mixing to be carried out immediately before administration, wherein the weight ratio between the two substances after mixing is comprised from 10:1 to 1:1 (D-ribose:creatine).

2. Kit according to claim 1 wherein D-ribose is in the form of powder.

3. Kit according to claim 2, wherein powdered D-ribose is mixed with an anti-agglomerating agent in an amount not exceeding 5%, preferably 2%.

4. Kit according to claim 1 wherein creatine is in the form of powder and D-ribose is in solution.

5. Kit according to claim 4, wherein D-ribose is kept with preservatives selected from the group consisting of benzoic acid derivatives and potassium sorbate.

6. Kit for extemporaneous preparation of a mixture comprising creatine and ribose, comprising a mixture of D-ribose, creatine, an aqueous solvent and appropriate instructions for solubilization of the mixture to be performed immediately before administration, wherein the weight ratio between the two substances in the mixture is comprised from 10:1 to 1:1 (D-ribose:creatine).

7. Kit according to claims 5-6 wherein such mixture includes a lubricating agent and an anti-agglomerating agent.

8. Kit according to claim 7 wherein the lubricating agent is magnesium stearate.

9. Kit according to claims 7-8 wherein the anti-agglomerating agent is silicon dioxide.

10. Extemporaneous composition of D-ribose and creatine in a ratio comprised from 10:1 to 1:1 (w/w) for use in reducing and promoting recovery from mild ischemic damage.

## Patentansprüche

1. Kit zur spontanen Zubereitung einer Kreatin und Ribose aufweisenden Mischung, umfassend zumindest einen Behälter mit D-Ribose, einen Behälter mit Kreatin und geeignete Instruktionen zur Solubilisierung und Mischung der beiden Komponenten und umgekehrt, wobei sich die Instruktionen auf das Mischen unmittelbar vor der Verabreichung beziehen, und wobei nach dem Mischen ein Gewichtsverhältnis der beiden Substanzen im Bereich 10 : 1 bis 1 : 1 ( D-Ribose : Kreatin) vorliegt.

2. Kit gemäß Anspruch 1, wobei die D-Ribose in Form von Pulver vorliegt.

3. Kit gemäß Anspruch 2, wobei die pulverförmige D-Ribose mit einer Menge von maximal 5%, vorzugsweise 2% eines Mittels gegen Verklumpung gemischt wird.

4. Kit gemäß Anspruch 1, wobei das Kreatin in Form von Pulver und die D-Ribose in Lösung vorliegt.

5. Kit gemäß Anspruch 4, wobei die D-Ribose mit Konservierungsstoffen aufbewahrt wird, ausgewählt aus der Gruppe von Benzoesäurederivaten und Kaliumsorbat.

6. Kit zur spontanen Zubereitung einer Kreatin und Ribose aufweisenden Mischung, aufweisend eine D-Ribose-Mischung, Kreatin, eine wässrige Lösung und geeignete Instruktionen zu Solubilisierung der Mischung, wobei sich die Instruktionen auf das Mischen unmittelbar vor der Verabreichung beziehen, und wobei in der Mischung ein Gewichtsverhältnis der beiden Substanzen im Bereich 10 : 1 bis 1 : 1 ( D-Ribose : Kreatin) vorliegt.

7. Kit gemäß Anspruch 5 bis 6, wobei eine solche Mischung ein Trennmittel und ein Mittel gegen Verklumpung umfasst.

8. Kit gemäß Anspruch 7, wobei das Trennmittel Magnesiumstearat ist.

9. Kit gemäß Anspruch 7 bis 8, wobei das Mittel gegen Verklumpung Silikondioxid ist.

10. Spontane Komposition von D-Ribose und Kreatin in einem Gewichtsverhältnis von 10 : 1 bis 1 : 1 zur Reduzierung und Erholung von leichten ischämischen Läsionen.

## Revendications

1. Kit pour la préparation extemporanée d'un mélange comprenant de la créatine et du ribose, incluant au moins un récipient avec du D-ribose, un récipient avec de la créatine et des instructions appropriées pour la solubilisation et le mélange, ou vice-versa, des deux composants, dans lequel lesdites instructions concernent le mélange devant être réalisé immédiatement avant l'administration, dans lequel le rapport en poids entre les deux substances après mélange va de 10:1 à 1:1 (D-ribose:créatine).

2. Kit selon la revendication 1, dans lequel le D-ribose se présente sous la forme de poudre.

3. Kit selon la revendication 2, dans lequel le D-ribose en poudre est mélangé à un agent anti-agglomérant dans une quantité ne dépassant pas 5 %, de préférence, 2 %.

4. Kit selon la revendication 1, dans lequel la créatine se présente sous la forme de poudre et le D-ribose est en solution.

5. Kit selon la revendication 4, dans lequel le D-ribose est conservé avec des conservateurs choisis dans le groupe constitué de dérivés de l'acide benzoïque et de sorbate de potassium.

6. Kit pour la préparation extemporanée d'un mélange comprenant de la créatine et du ribose, comprenant un mélange de D-ribose, de créatine, d'un solvant aqueux et des instructions appropriées pour que la solubilisation du mélange soit effectuée immédiatement avant administration, dans lequel le rapport en poids entre les deux substances dans le mélange va de 10:1 à 1:1 (D-ribose:créatine).

7. Kit selon les revendications 5-6 dans lequel un tel mélange inclut un agent lubrifiant et un agent anti-agglomérant.

8. Kit selon la revendication 7 dans lequel l'agent lubrifiant est du stéarate de magnésium.

9. Kit selon les revendications 7-8 dans lequel l'agent anti-agglomérant est du dioxyde de silicium.

10. Composition extemporanée de D-ribose et de créatine dans un rapport allant de 10:1 à 1:1 (P/P) destinée à être utilisée pour réduire et favoriser le rétablissement d'une lésion ischémique bénigne.
